(19) Europäisches Patentamt
European Patent Office
Office européen des brevets

(11) Veröffentlichungsnummer: **0 104 387**
**A1**

(12) # EUROPÄISCHE PATENTANMELDUNG

(21) Anmeldenummer: **83107924.9**

(22) Anmeldetag: **11.08.83**

(51) Int. Cl.$^3$: **C 07 J 1/00**
A 61 K 31/565

(30) Priorität: **24.08.82 DE 3231827**

(43) Veröffentlichungstag der Anmeldung:
**04.04.84 Patentblatt 84/14**

(84) Benannte Vertragsstaaten:
**AT BE CH DE FR GB IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65(DE)**

(72) Erfinder: **Neef, Günter, Dr.**
**Darmstädter Strasse 9**
**D-1000 Berlin 15(DE)**

(72) Erfinder: **Eder, Ulrich, Dr.**
**Zeltinger Strasse 17**
**D-1000 Berlin 28(DE)**

(72) Erfinder: **Beier, Sybille, Dr.**
**Uhlandstrasse 121**
**D-1000 Berlin 31(DE)**

(72) Erfinder: **Vierhufe, Harry**
**Köpenicker Strasse 6 A**
**D-1000 Berlin 36(DE)**

(72) Erfinder: **Wiechert, Rudolf, Prof. Dr.**
**Petzower Strasse 8 A**
**D-1000 Berlin 39(DE)**

(72) Erfinder: **Elger, Walter, Dr.**
**Schorlemer Allee 12 B**
**D-1000 Berlin 33(DE)**

(72) Erfinder: **Schillinger, Ekkehard Dr.**
**Im Amseltal 50**
**D-1000 Berlin 28(DE)**

(54) **11-Beta-Aryl-17-alpha-alkinyl-17-beta-hydroxy-4,9(10)-estradien-3-on-Derivate, deren Herstellung und diese enthaltende pharmazeutische Präparate.**

(57) Es werden
11β-Aryl-17α-alkinyl-17β-hydroxy-4.9(10)-estradien-3-on-Derivate der allgemeinen Formel I

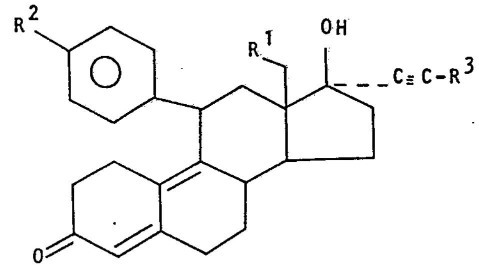

(I),

worin

$R^1$ ein Wasserstoffatom oder eine $C_1-C_3$-Alkylgruppe darstellt,

$R^2$ eine freie oder acylierte Hydroxygruppe, eine $C_1-C_3$-Alkoxygruppe oder Allyloxygruppe darstellt und

$R^3$ ein Wasserstoffatom, ein Chloratom oder eine Methylgruppe bedeuten, wobei
$R^2$ keine Methoxygruppe bedeutet, wenn
$R^1$ und $R^3$ gleichzeitig ein Wasserstoffatom darstellen,

beschrieben, die starke antigestagene Wirkung besitzen und zur postcoitalen Fertilitätskontrolle verwendet werden können.

EP 0 104 387 A1

Die Erfindung betrifft neue 11ß-Aryl-17α-alkinyl-17ß-hydroxy-4.9(10)-estradien-3-on-derivate der allgemeinen Formel I, ein Verfahren zu deren Herstellung und diese Verbindungen enthaltende pharmazeutische Präparate.

In 11ß-Stellung substituierte 17α-Alkinyl-4.9(10)-estradien-Verbindungen sind bekannt. So werden beispielsweise die 11ß-substituierten 17α-Ethinylverbindungen dieser Stoffklasse von A. Bélanger u.a. in Steroids 37(1981) S.361 ff. beschrieben und über die 17α-Propinylverbindung der Formel A

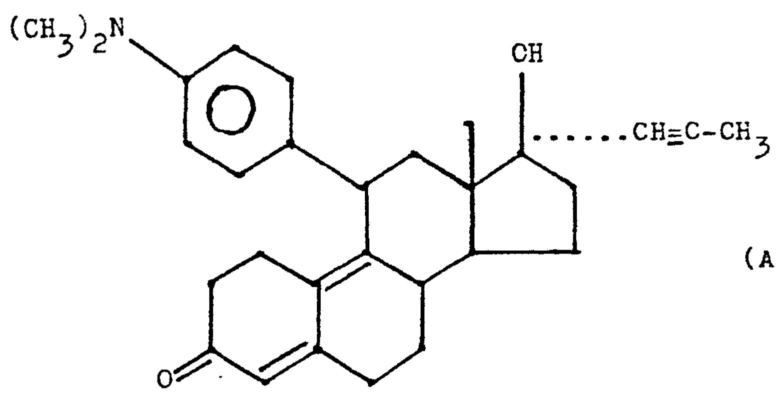

(A) ,

wurde kürzlich ( 46th Annual Meeting of the Endocrine Society, San Francisco (Ca.), June 16-18, 1982) berichtet.

Diese Verbindungen besitzen eine starke Affinität zum Gestagenrezeptor ohne selbst gestagene Aktivität zu besitzen. Sie sind kompetitive Antagonisten des Progesterons (Anti-Gestagene) und sind zur Auslösung von Aborten geeignet, da sie das zur Aufrechterhaltung der Schwangerschaft erforderliche Progesteron vom Rezeptor verdrängen. Sie sind deshalb wertvoll und interessant im Hinblick auf ihre Verwendung zur postcoitalen Fertilitätskontrolle.

Es wurde gefunden, daß die neuen Verbindungen der allgemeinen Formel I

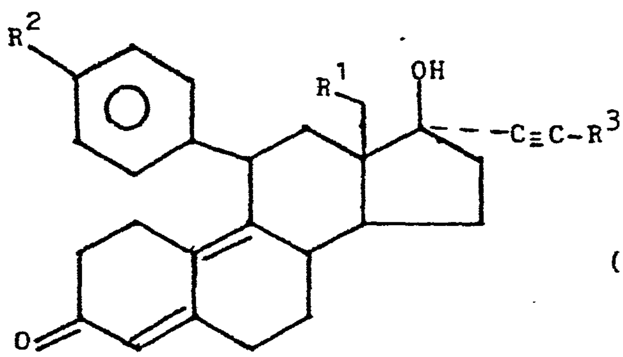

(I) ,

worin

$R^1$ ein Wasserstoffatom oder eine $C_1$-$C_3$-Alkylgruppe darstellt,

$R^2$ eine freie oder acylierte Hydroxygruppe, eine $C_1$-$C_3$-Alkoxygruppe oder Allyloxygruppe darstellt und

$R^3$ ein Wasserstoffatom, ein Chloratom oder eine Methylgruppe bedeuten, wobei $R^2$ keine Methoxygruppe bedeutet, wenn $R^1$ und $R^3$ gleichzeitig ein Wasserstoffatom darstellen

ebenfalls antigestagene Wirksamkeit besitzen, im Gegensatz zu den bisher bekannten Verbindungen dieser Stoffklasse aber keine antiglucocorticoiden (Europäische Patentanmeldung 82.400025.1, Publikations-Nr. 0 057 115), sondern glucocorticoide Nebenwirkungen zeigen.

Während die erfindungsgemäßen Verbindungen die schockprotektive Wirkung der endogenen Glucocorticoide erhöhen, wird dieser Schutz durch die Verbindungen des Standes der Technik beeinträchtigt.

Die Verbindungen können in Form pharmazeutischer Präparate Verwendung finden. Die Herstellung der Präparate erfolgt nach an sich bekannten Methoden der Galenik durch Mischen mit einem für die enterale, perkutane oder parenterale Applikation geeigneten pharmazeutischen, organischen oder anorganischen inerten Trägermaterial.

Die Dosierung der erfindungsgemäßen Wirkstoffe liegt beim Menschen bei 10 bis 1000 mg pro Tag.

Die im Rest $R^1$ und in der $C_1$-$C_3$-Alkoxygruppe $R^2$ enthaltenenen Alkylgruppen sind die Methyl-, Äthyl- oder Propylgruppe, wobei die Methylgruppe bevorzugt ist.

Ist $R^2$ eine acylierte Hydroxylgruppe, so leitet sich der Acylrest von einer organischen Carbonsäure ab, die 1 bis 3 Kohlenstoffatome im Alkylteil besitzt, wobei der Acetylrest bevorzugt ist.

Die neuen Verbindungen der Formel I werden erfindungsgemäß nach dem Verfahren gemäß Anspruch 7 hergestellt. Das erfindungsgemäße Verfahren wird in einem wasserfreien aprotischen Lösungsmittel wie Tetrahydrofuran (THF), Dioxan oder Diäthyläther durchgeführt, indem man Magnesiumspäne mit dem Arylhalogenid $R^{2'}-C_6H_4-X$ (mit $R^{2'}$ und X in der oben angegebenen Bedeutung), falls erforderlich in Gegenwart von Methyljodid als Katalysator, bei 20°C bis 40°C, vorzugsweise bis 35°C, zur Grignardverbindung umsetzt. Nach beendeter Reaktion setzt man der erhaltenen Grignard-Lösung bei 0°C bis 10°C, vorzugsweise bei 0°C bis 5°C, die entsprechende Menge Kupfer-I-chlorid zu, rührt die Reaktionsmischung 10 bis 20 Minuten unter Eiskühlung und gibt die Lösung der Ausgangsverbindung der allgemeinen Formel II, gelöst in einem geeigneten wasserfreien aprotischen Lösungsmittel wie Tetrahydrofuran (THF), Dioxan oder Diäthyläther, vorzugsweise THF, zu und rührt 10 bis 20 Stunden, vorzugsweise 16 Stunden bei 20°C und arbeitet die Reaktionsmischung auf. Der genaue Zeitpunkt des Reaktionsendes wird vorzugsweise durch Dünnschichtchromatographie bestimmt.

Das so erhaltene Rohprodukt der allgemeinen Formel IV, das die 3-Ketalgruppe und eine 5α-Hydroxygruppe enthält, wird nach bekannten Verfahren in einem mit Wasser mischbaren Lösungsmittel wie wäßrigem Ethanol oder Methanol oder Aceton gelöst und die Lösung in Gegenwart von ketalytischen Mengen Mineralsäure oder Sulfonsäure wie Salzsäure, Schwefelsäure, Perchlorsäure oder p-Toluolsulfonsäure solange zum Sieden erhitzt, bis die Ketalspaltung und Wasserabspaltung und die Abspaltung zusätzlicher, gegebenenfalls vorhandener säureempfindlicher Schutzgruppen beendet sind, was durch Dünnschichtchromatographie ermittelt wird. Nach Aufarbeitung und Reinigung erhält man die erfindungsgemäßen Verbindungen der allgemeinen Formel I. Stellt der Rest $R^2$ eine freie Hydroxylgruppe dar, so kann diese gewünschtenfalls unter Erhalt der freien Hydroxylgruppe in 17ß-Stellung verestert werden, indem man beispielsweise nach bekannten Verfahren mit einem Carbonsäureanhydrid, wie beispielsweise Essigsäureanhydrid oder Propionsäureanhydrid, in einem geeigneten Lösungsmittel wie Dimethylformamid (DMF) oder Pyridin oder in DMF in Gegenwart von Blei-(II)-acetat oder -carbonat bei einer Temperatur von 20°C bis 50°C umsetzt, bis die dünnschichtchromatographische Kontrolle das Ende der Acylierung anzeigt.

Die zur Durchführung des erfindungsgemäßen Verfahrens benötigten Ausgangsverbindungen der allgemeinen Formel II

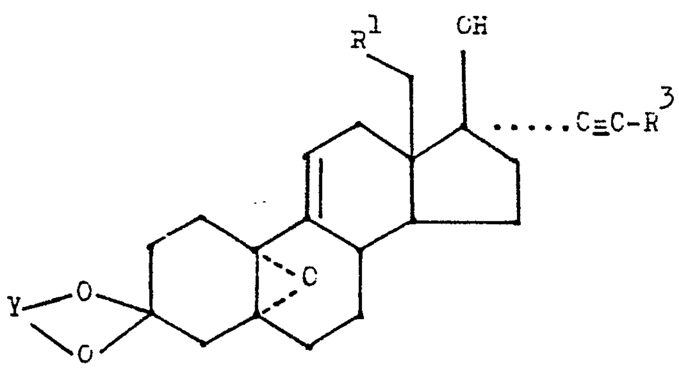

(II) ,

worin $R^1$, $R^3$ und Y die obengenannten Bedeutungen haben, werden aus dem bekannten 4,9(10)-Estradien-3,17-dion der allgemeinen Formel V

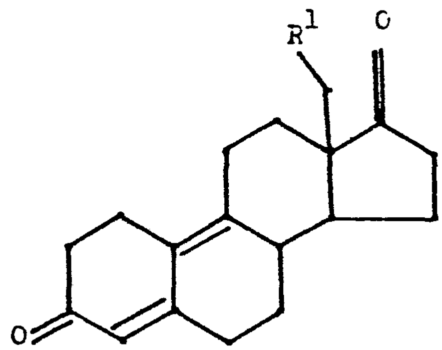

(V) ,

worin $R^1$ die obengenannte Bedeutung besitzt,hergestellt, indem man nach dem Fachmann bekannten Verfahren die 3-Ketogruppe mit einem geeigneten Alkylendiol wie Ethylenglykol oder 2.2-Dimethylpropandiol in Gegenwart eines wasserentziehenden Mittels wie Orthoameisensäuretrimethylester und einer Mineral- oder Sulfonsäure partiell ketalisiert zur Verbindung der allgemeinen Formel VI

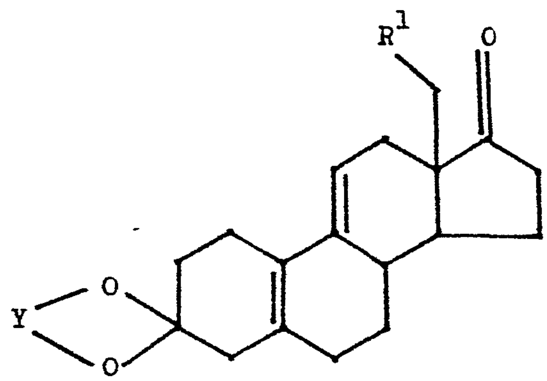

(VI) ,

worin $R^1$ und Y die obengenannten Bedeutungen haben. Die Verbindungen der allgemeinen Formel VI werden nun nach dem Fachmann bekannten Verfahren unter Erhalt der Ketalfunktion Y partiell epoxidiert zum 5(10)-Epoxid der allgemeinen Formel VII

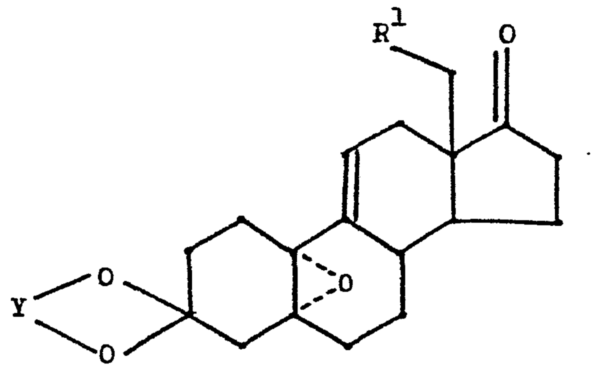

(VII) ,

worin $R^1$ und Y die obengenannten Bedeutungen haben.

Die Alkinylierung der 17-Ketogruppe zu den Ausgangsverbindungen der Formel II wird unter Beibehaltung aller übrigen Funktionen der Verbindungen der allgemeinen Formel VII partiell nach bekannten Methoden mit einer metallorganischen Ethinyl-, Chlorethinyl- oder Propinyl-verbindung durchgeführt. Solche metallorganischen Verbindungen sind zum Beispiel Alkalimetallacetylide, wie zum Beispiel Kalium- und Lithiumacetylid, -chloracetylid bzw. -methylacetylid.

Es ist für den Fachmann überraschend, daß die 17-Alkinylierung der Verbindungen der Formel VII möglich ist, ohne daß die 5α,10α-Oxidogruppe angegriffen wird.

Anhand der Herstellungsvorschriften für 17α-Ethinyl-3,3-(2,2-Dimethylpropylen-1,3-dioxy)-5α,10α-epoxy-18-methyl-9(11)-estren-17ß-ol sowie der entsprechenden 17α-Propinylverbindung soll die Herstellung von Ausgangsverbindungen der allgemeinen Formel II näher erläutert werden.

A) 17α-Ethinyl-3,3-(2,2-dimethylpropylen-1,3-dioxy)-5α,10α-epoxy-18-methyl-9(11)-estren-17ß-ol

1.) Eine Lösung von 20 g 18-Methyl-4,9(10)-estradien-3,17-dion in 200 ml Methylenchlorid wird nach Zusatz von 17,8 g 2,2-Dimethylpropan-1,3-diol, 10 ml Orthoameisensäuretrimethylester und 30 mg p-Toluolsulfonsäure 16 Stunden bei 25°C gerührt. Anschließend wird sukzessive mit gesättigter $NaHCO_3$-Lösung und mit Wasser gewaschen, über Natriumsulfat getrocknet und im Wasserstrahlvakuum eingeengt. Kristallisation des Rohproduktes aus Diisopropyläther/Hexan ergibt 25,5 g (97,8 %) 3,3-(2,2-Dimethylpropylen-1,3-dioxy)-18-methyl-5(10),9(11)-estradien-17-on vom Schmelzpunkt 113-115°C.

2.) Eine Lösung des unter 1.) beschriebenen Monoketals (42 g) in 420 ml Methylenchlorid und 0,3 ml Pyridin wird unter Eiswasserkühlung zunächst mit 1,6 ml Hexachloraceton, dann tropfenweise mit 17,2 ml $H_2O_2$ (30 %) versetzt. Man rührt anschließend 20 Stunden bei Raumtemperatur, wäscht mit 5-proz. $Na_2S_2O_3$-Lösung und gesättigter Kochsalzlösung, trocknet über $Na_2SO_4$ und engt ein. Das so erhaltene Rohprodukt wird zur Abtrennung des unerwünschten 5ß,10ß-Epoxids über neutralem Aluminiumoxid mit Hexan/Essigester chromatographiert. Kristallisation der Hauptfraktion aus Essigester/Diisopropylether ergibt 23,2 g (52,9 %) 3,3-(2,2-Dimethylpropylen-1,3-dioxy)-5α,10α-epoxy-18-methyl-9(11)-estren-17-on vom Schmelzpunkt 156-158°C.

3.) Über Lithiumaluminiumhydrid destilliertes Tetrahydrofuran (275 ml) wird 30 Minuten bei Eiswasserkühlung mit Acetylen gesättigt. Dazu tropft man bei 0°C 50,3 ml einer 1,2-molaren Lösung von n-Butyllithium in Hexan. Anschließend erfolgt tropfenweise Zugabe einer Lösung von 5,0 g des unter 2.) erhaltenen Epoxids in 50 ml absolutem THF. Man rührt 3 Stunden bei Raumtemperatur nach, gießt in $NH_4Cl$-Lösung und extrahiert mit Essigester. Das so erhaltene 17α-Ethinyl-3,3-(2,2-dimethyl propylen-1,3-dioxy)-5α,10α-epoxy-18-methyl-9(11)-estren-17ß-ol wird als Rohprodukt in die erfindungsgemäße Reaktion eingesetzt.

B) 3,3- (2,2-Dimethylpropylen-1,3-dioxy) -5α,10α-epoxy-18-methyl-17α-propinyl -9(11)-estren-17ß-ol

500 ml absolutes THF werden innerhalb von 30 Minuten unter Eiswasserkühlung mit Methylacetylen gesättigt. Anschließend tropft man 100 ml einer 1,2-molaren Lösung von n-Butyllithium in Hexan hinzu. Es wird 15 Minuten unter Eiskühlung nachgerührt, dann eine Lösung von 9,2 g 3,3-(2,2-Dimethylpropylen-1,3-dioxy)-5α,10α-epoxy-18-methyl-9(11)-estren-17-on in 95 ml absolutem THF zur Reaktionssuspension getropft. Nach 3-stündigem Rühren bei +5°C gießt man in wässrige $NH_4$-Cl-Lösung und extrahiert mit Essigester. Man erhält nach Kristallisation des Rohproduktes aus Ethanol 8,4 g 3,3-(2,2-Dimethylpropylen-1,3-dioxy)-5α, 10α-epoxy-18-methyl-17α-propinyl-9(11)-estren-17ß-ol vom Schmelzpunkt 174-176°C.

Beispiel 1

17α-Ethinyl-17ß-hydroxy-11ß-(4-methoxyphenyl)-18-methyl-4,9(10)-estradien-3-on

A.) Magnesiumspäne (2,4 g) werden in 120 ml absolutem THF suspendiert. Dazu tropft man 11,6 ml 4-Bromanisol so zu, daß die Innentemperatur 35°C nicht übersteigt. 60 ml der so erhaltenen Grignard-Lösung werden entnommen und unter Eiskühlung mit 260 mg CuCl versetzt. Man rührt 15 Minuten, ehe man eine Lösung von 4,5 g 17α-Ethinyl-3,3-(2,2-dimethylpropylen-1,3-dioxy)-5α,10α-epoxy-18-methyl-9(11)-estren-17ß-ol in 3o ml absolutem THF bei 0°C zutropft. Anschließend wird 16 Stunden bei Raumtemperatur gerührt, danach in Eiswasser gegossen mit mit Methylenchlorid extrahiert. Das ölige Rohprodukt [17α-Ethinyl-3,3-(2,2-dimethylpropylen-1,3-dioxy)-11ß-(4-methoxyphenyl)-18-methyl-9(10)-estren-5α,17ß-diol] wird ohne Aufreinigung in die Folgestufe eingesetzt.

B.) Das erhaltene Rohprodukt wird in 125 ml 90-%igem wäßrigem Ethanol gelöst und nach Zugabe von 1,1 g p-Toluolsulfonsäure 15 Minuten unter Rückfluß erhitzt. Danach gießt man in Wasser und extrahiert mit Methylenchlorid. Chromatographie des Produkts über Kieselgel mit Hexan/Essigester ergibt 3,5 g 17α-Ethinyl-17ß-hydroxy-11ß-(4-methoxyphenyl)-18-methyl-4,9(10)-estradien-3-on vom Schmelzpunkt 213-215°C (Essigester).

Beispiel 2

17α-Ethinyl-17ß-hydroxy-11ß-(4-hydroxyphenyl)-18-methyl-4,9(10)-estradien-3-on

Unter den Bedingungen des Beispiels 1 A.) wird aus p-Tetrahydropyranyloxy-phenylbromid (15,7 g) und 1,7 g Magnesium ein Grignard-Reagenz hergestellt und mit 4,8 g 17α-Ethinyl-3,3-(2,2-dimethylpropylen-1,3-dioxy)-5α,10α-epoxy-18-methyl-9(11)-estren-17ß-ol umgesetzt. Nach Behandlung des so erhaltenen Rohproduktes mit p-Toluolsulfonsäure und Aufarbeitung analog Beispiel 1 B.) erhält man 4,25 g der Titelverbindung vom Schmelzpunkt 287-289°C.

Beispiel 3

11ß-(4-Acetoxyphenyl)-17α-ethinyl-17ß-hydroxy-18-methyl-4,9(10)-estradien-3-on

Eine Lösung von 1,5 g der nach Beispiel 2 erhaltenen Verbindung in 14 ml Dimethylformamid und 3 ml Essigsäureanhydrid wird nach Zusatz von 150 mg Blei(II)-acetat 4 Stunden bei 50°C gerührt. Anschließend verdünnt man mit Essigester, wäscht mit Wasser und gesättigter $NaHCO_3$-Lösung, trocknet über $Na_2SO_4$ und engt ein. Der Rückstand wird nach Filtration über Kieselgel aus Essigester/Diisopropyläther kristallisiert. Man erhält 1,4 g der Titelverbindung vom Schmelzpunkt 202-203°C.

Beispiel 4

17α-Ethinyl-11ß-(4-alloxyphenyl)-17ß-hydroxy-4,9-estradien-3-on

A.) Zu einer Suspension von 1,8 g Magnesiumspänen in 100 ml absolutem THF tropft man 13,0 g 4-Alloxybrombenzol so zu, daß die Temperatur der Reaktionslösung 35 °C nicht übersteigt. Nach dem Abkühlen auf 0 °C gibt man 400 mg Kupfer(I)chlorid hinzu und rührt 15 Minuten bei 0 °C, bevor eine Lösung von 3,0 g 3,3-(2,2-Dimethyl-propan-1,3-dioxy)17α-ethinyl-5α,10α-epoxy-9-estren-17ß-ol in 50 ml absolutem THF tropfenweise zugegeben wird. Anschließend rührt man 4 Stunden bei Raumtemperatur und arbeitet wie unter Beispiel 1 A auf.

B.) Das so erhaltene Rohprodukt wird in 56 ml 90 %igem Ethanol gelöst und nach Zusatz von 550 mg p-Toluolsulfonsäure 10 Minuten unter Rückfluß erhitzt. Nach dem Abkühlen gießt man in Eiswasser und extrahiert mit Essigester. Nach Chromatographie an Kieselgel mit Hexan/Essigester erhält man 1,4 g 17α-Ethinyl-11ß-(4-alloxyphenyl)-17ß-hydroxy-4,9-estradien-3-on als gelbliches Glas.
$^1$H-NMR ($CDCl_3$): δ = 0,55 ppm (s,3H,H-18);
2,63 (s,1H,C≡CH); 4,37 (m,1H,H-11);
4,49 (m,2H,$OCH_2$); 5,76 (s,1H,H-4);
5,16-5,50 u. 5,81-6,28 (m,3H,$CH=CH_2$);
6,79 u. 7,06 (AA'BB', je 2H, aromat. H).

Beispiel 5

11ß-(4-Alloxyphenyl)-17ß-hydroxy-17α-(1-propinyl)-4,9-estradien-3-on

A.) 150 ml absolutes THF werden bei 0 °C mit Methylacetylen gesättigt. Dazu tropft man 25,2 ml einer 15 %igen Lösung von n-Butyllithium in Hexan und rührt 15 Minuten bei 0 °C, ehe eine Lösung von 1,2 g 3,3-(2,2-Dimethyl-propan-1,3-dioxy)-5α,10α-epoxy-9(11)-estren-17-on in 12 ml absolutem THF tropfenweise hinzugegeben wird. Anschließend rührt man 2 Stunden bei Raumtemperatur, gießt in Eiswasser und extrahiert mit Essigester. Das ölige Rohprodukt (1,30 g) wird ohne weitere Reinigung umgesetzt.

B.) Aus 0,9 g Magnesium, 6,5 g 4-Alloxy-brombenzol und 200 mg CuCl stellt man unter den Bedingungen des Beispiels 4 A.) ein Grignard-Reagenz her und setzt mit 1,3 g 3,3-(2,2-Dimethyl-propan-1,3-dioxy)-5α,10α-epoxy-17α-(1-propinyl)-9(11)-estren-17ß-ol (Rohprodukt) um. Durchführung und Aufarbeitung erfolgen analog Beispiel 4 A). Man erhält nach Chromatographie über $Al_2O_3$ mit Hexan/Essigester und nach Kristallisation der Hauptfraktion aus Essigester/Diisopropylether 1,15 g 11ß-(4-Alloxyphenyl)-3,3-(2,2-dimethyl-propan-1,3-dioxy)-17α-(1-propinyl)-9(10)-estren-5α,17ß-diol vom Schmelzpunkt 193 - 197 °C.

C.) Eine Lösung von 0,8 g des unter B.) erhaltenen Produkts in 20 ml 90 %igem Ethanol wird nach Zusatz von 130 mg p-Toluolsulfonsäure 15 Minuten unter Rückfluß erhitzt. Nach Aufarbeitung und Chromatographie analog Beispiel 4 B.) erhält man 400 mg 11ß-(4-Alloxyphenyl)-17ß-hydroxy-17α-(1-propinyl)-4,9-estradien-3-on als festen Schaum.
$^1$H-NMR ($CDCl_3$): δ = 0,54 ppm (s,3H,H-18);
1,88 (s,3H,C≡C-$CH_3$); 4,37 (m,1H,H-11);
4,49 (m,2H,$OCH_2$); 5,77 (s,1H,H-4);
5,15-5,52 u. 5,80-6,29 (m,3H,CH=$CH_2$);
6,80 u. 7,04 (AA'BB', je 2H, aromat.H).

Patentansprüche für die Vertragsstaaten BE, CH, DE, FR, GB, IT, LU, NL, SE

1.) 11ß-Aryl-17α-alkinyl-17ß-hydroxy-4.9(10)-estradien-3-on-Derivate der allgemeinen Formel I

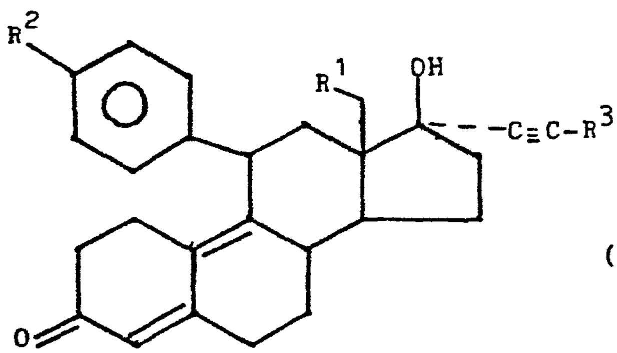

(I) ,

worin

$R^1$ ein Wasserstoffatom oder eine $C_1$-$C_3$-Alkylgruppe

$R^2$ eine freie oder acylierte Hydroxygruppe, eine $C_1$-$C_3$-Alkoxygruppe oder Allyloxygruppe darstellt und

$R^3$ ein Wasserstoffatom, ein Chloratom oder eine Methylgruppe bedeuten, wobei $R^2$ keine Methoxygruppe bedeutet, wenn $R^1$ und $R^3$ gleichzeitig ein Wasserstoffatom darstellen.

2.) 17α-Ethinyl-17ß-hydroxy-11ß-(4-methoxyphenyl)-18-methyl-4,9(10)-estradien-3-on.

3.) 17α-Ethinyl-17ß-hydroxy-11ß-(4-hydroxyphenyl)-18-methyl-4,9(10)-estradien-3-on.

4.) 11ß-(4-Acetoxyphenyl)-17α-ethinyl-17ß-hydroxy-18-methyl-4,9(10)-estradien-3-on.

5.) 17α-Ethinyl-11ß-(4-alloxyphenyl)-17ß-hydroxy-4,9-estradien-3-on.

6.) 11ß-(4-Alloxyphenyl)-17ß-hydroxy-17α-(1-propinyl)-4,9-estradien-3-on.

7.) Verfahren zur Herstellung von 11ß-Aryl-17α-alkinyl-17ß-hydroxy-4,9(10)-estradien-3-on-Derivaten der allgemeinen Formel I

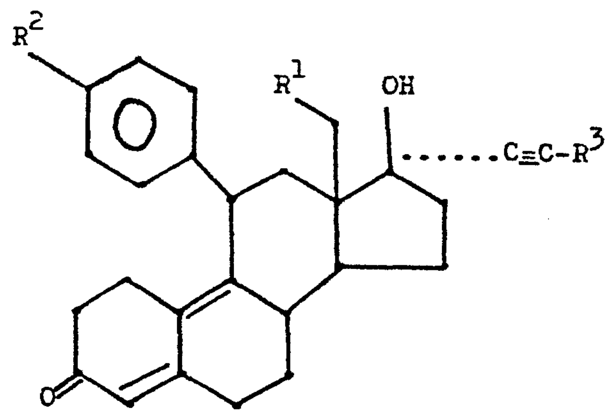

(I) ,

worin

$R^1$ ein Wasserstoffatom oder eine $C_1$-$C_3$-Alkylgruppe

$R^2$ eine freie oder acylierte Hydroxygruppe, eine $C_1$-$C_3$-Alkoxygruppe oder Allyloxygruppe darstellt und

$R^3$ ein Wasserstoffatom, ein Chloratom oder eine Methylgruppe bedeuten, wobei $R^2$ keine Methoxygruppe bedeutet, wenn $R^1$ und $R^3$ gleichzeitig ein Wasserstoffatom darstellen,

dadurch gekennzeichnet, daß man in an sich bekannter Weise eine Verbindung der allgemeinen Formel II

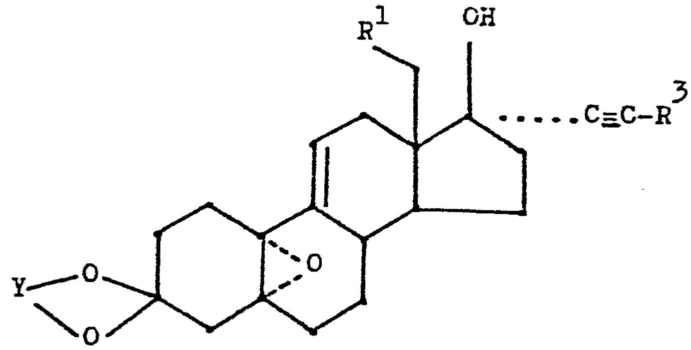

(II) ,

worin $R^1$ und $R^3$ die oben angegebenen Bedeutungen haben und

Y eine Äthylen- oder 2,2-Dimethylpropylengruppe darstellt,

mit einer Grignard-Verbindung der allgemeinen Formel III

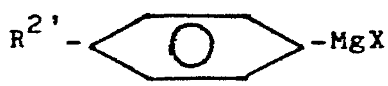

(III) ,

worin $R^{2'}$ die oben genannte Bedeutung für $R^2$ besitzt, vorhandene Hydroxygruppen jedoch in geschützter Form vorliegen können und

X ein Chlor- oder Bromatom darstellt,

in Gegenwart von CuCl umsetzt zum Zwischenprodukt der allgemeinen Formel IV

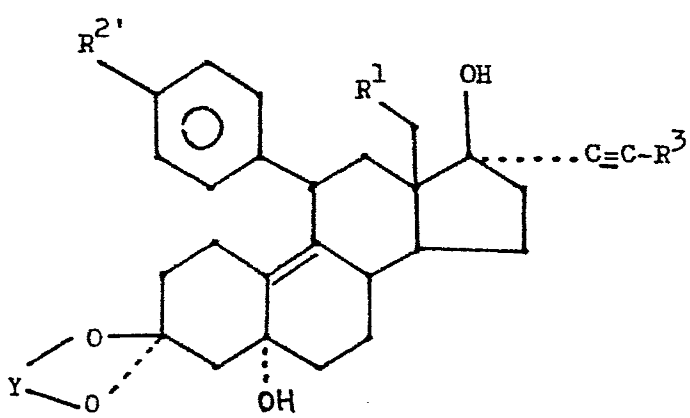

(IV),

worin $R^1$ bis $R^3$ und Y die oben angegebenen Bedeutungen haben, und das Zwischenprodukt der allgemeinen Formel IV zur Spaltung des 3-Ketalschutzes, zur Beseitigung weiterer gegebenenfalls vorhandener mit Säure abspaltbarer Schutzgruppen und zur Wasserabspaltung unter gleichzeitiger Ausbildung der 4(5)- Doppelbindung mit Säure behandelt und gewünschtenfalls die im Rest $R^2$ vorhandene Hydroxygruppe verestert.

8.) Pharmazeutische Präparate, gekennzeichnet durch einen Gehalt an einer Verbindung gemäß Anspruch 1 bis 6.

0104387



Österreich

Patentanspruch für den Vertragsstaat AT

Verfahren zur Herstellung von 11ß-Aryl-17α-alkinyl-17ß-hydroxy-4,9(10)-estradien-3-on-Derivaten der allgemeinen Formel I

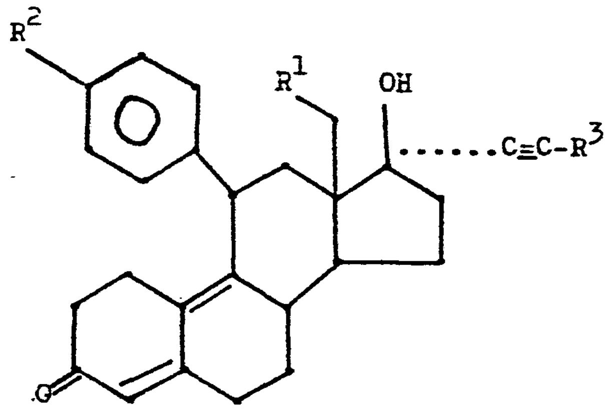

(I) ,

worin

$R^1$ ein Wasserstoffatom oder eine $C_1$-$C_3$-Alkylgruppe

$R^2$ eine freie oder acylierte Hydroxygruppe, eine $C_1$-$C_3$-Alkoxygruppe oder Allyloxygruppe darstellt und

$R^3$ ein Wasserstoffatom, ein Chloratom oder eine Methylgruppe bedeuten, wobei $R^2$ keine Methoxygruppe bedeutet, wenn $R^1$ und $R^3$ gleichzeitig ein Wasserstoffatom darstellen,

dadurch gekennzeichnet, daß man in an sich bekannter Weise eine Verbindung der allgemeinen Formel II

Österreich

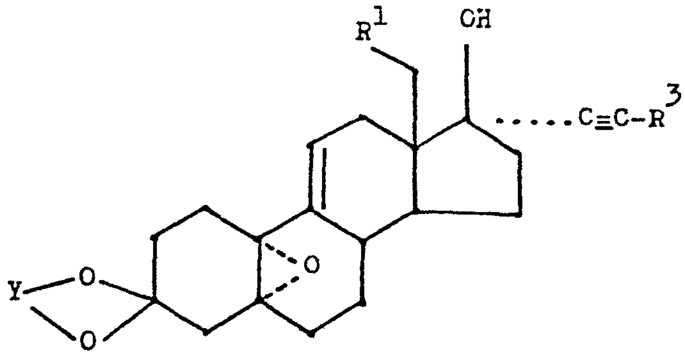 (II) ,

worin $R^1$ und $R^3$ die oben angegebenen Bedeutungen haben und

Y eine Äthylen- oder 2,2-Dimethylpropylengruppe darstellt,

mit einer Grignard-Verbindung der allgemeinen Formel III

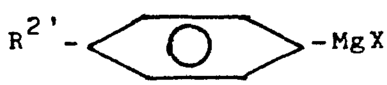 (III) ,

worin $R^{2'}$ die oben genannte Bedeutung für $R^2$ besitzt, vorhandene Hydroxygruppen jedoch in geschützter Form vorliegen können und

X ein Chlor- oder Bromatom darstellt,

in Gegenwart von CuCl umsetzt zum Zwischenprodukt der allgemeinen Formel IV

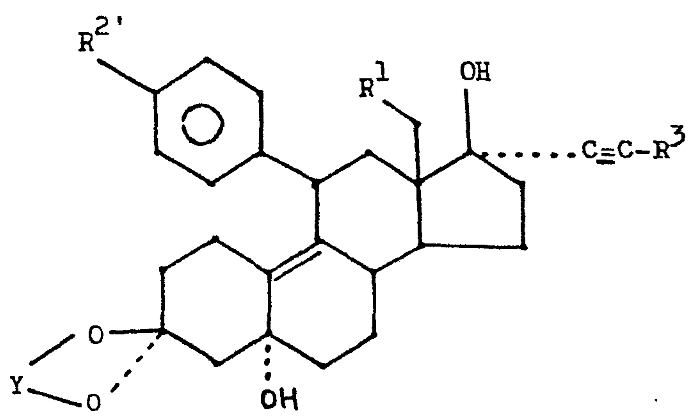 (IV),

 Österreich

worin $R^1$ bis $R^3$ und Y die oben angegebenen Bedeutungen haben, und das Zwischenprodukt der allgemeinen Formel IV zur Spaltung des 3-Ketalschutzes, zur Beseitigung weiterer gegebenenfalls vorhandener mit Säure abspaltbarer Schutzgruppen und zur Wasserabspaltung unter gleichzeitiger Ausbildung der 4(5)- Doppelbindung mit Säure behandelt und gewünschtenfalls die im Rest $R^2$ vorhandene Hydroxygruppe verestert.

0104387

Europäisches Patentamt

# EUROPÄISCHER RECHERCHENBERICHT

Nummer der Anmeldung

EP 83 10 7924

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X,Y | FR-A-2 377 418 (ROUSSEL-UCLAF) * Ansprüche * | 1,7,8 | C 07 J 1/00<br>A 61 K 31/565 |
| X,Y | STEROIDS, Band 37, Nr. 4, April 1981, Seiten 361-382, Holden-Pay, San Francisco, US A. BELANGER u.a.: "Regio and sterospecific synthesis of 11beta-substituted 19-norsteroids" * Insgesamt * | 1,7,8 | |
| Y | EP-A-0 057 115 (ROUSSEL-UCLAF) * Ansprüche * | 1,7,8 | |
| E | DE-A-3 307 143 (ROUSSEL-UCLAF) * Ansprüche 1,5,9-11 * | 1,7,8 | |
| | | | RECHERCHIERTE SACHGEBIETE (Int. Cl. 3) |
| | | | C 07 J 1/00 |

Der vorliegende Recherchenbericht wurde für alle Patentansprüche erstellt.

| Recherchenort | Abschlußdatum der Recherche | Prüfer |
|---|---|---|
| DEN HAAG | 29-11-1983 | HENRY J.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument